# EUROPEAN PATENT APPLICATION

(11) **EP 4 529 855 A1**
(43) Date of publication of application: **02.04.2025**
(21) Application number: 24203218.3
(22) Date of filing: 27.09.2024
(51) Int. Cl.: A61B 8/06, A61B 8/08, G06T 7/00

(54) **ULTRASONIC DIAGNOSTIC APPARATUS AND MEDICAL INFORMATION PROCESSING METHOD**

(30) Priority: 27.09.2023 JP 2023164256; 18.12.2023 JP 2023213405; 18.12.2023 JP 2023213406; 04.03.2024 JP 2024032436; 25.09.2024 JP 2024166275
(71) Applicant: Canon Medical Systems Corporation, Tochigi 324-0036 (JP)
(72) Inventor: USUMURA, Masashi, Otawara-shi, 324-0036 (JP); TAKAHASHI, Hiroki, Otawara-shi, 324-0036 (JP)
(74) Representative: Marks & Clerk LLP

(57) **Abstract**

An ultrasonic diagnostic apparatus (10) of an embodiment includes an ultrasonic probe (5), an acquisition unit (113), an extraction unit (114), and an output unit (116). The ultrasonic probe (5) transmits an ultrasonic wave to a subject, and receives the ultrasonic wave reflected by the subject. The acquisition unit (113) acquires first ultrasonic data of the subject, based on the ultrasonic wave received by the ultrasonic probe (5). The extraction unit (114) extracts identification information that identifies a position of a local peak for each predetermined region of a frame represented by the first ultrasonic data. The output unit (116) outputs second ultrasonic data, based on the identification information extracted by the extraction unit (114) and ultrasonic data representing an object to be processed.

## Description

### FIELD

Embodiments described herein relate generally to an ultrasonic diagnostic apparatus, a medical information processing apparatus, and a medical information processing method.

### BACKGROUND

Conventionally, techniques for improving the image quality of an ultrasonic image including fine flow paths and fluid (for example, blood flow, contrast agent, and the like) have been known. For example, Patent Document 1 (Japanese Patent Application Laid-open No. 2016-20881) discloses a technique for generating an image representing the boundary of the flow paths (for example, shape of blood vessel), by removing clutter in continuous ultrasonic reception signals within a predetermined time, and averaging the signals of the fluid from which clutter is removed within a predetermined time. Moreover, Patent Document 2 (Japanese Unexamined Patent Application Publication No. 2019-526350) discloses a technique for performing super-resolution processing that improves the resolution (pixel density) of an ultrasonic image, and that sharpens the peak of a speckle pattern in the ultrasonic image.

However, with the technique of Patent Document 1, when the fineness of a flow path increases, the speckle pattern does not fit in the flow path due to the resolution limit of the ultrasonic diagnostic apparatus, and the image of the flow path cannot be displayed at high quality. Moreover, with the technique of Patent Document 2, it is difficult to generate an image on the operation of an ultrasonic probe, because the computational load of the super-resolution processing is high (bad time resolution).

### SUMMARY OF INVENTION

### (Note 1)

A medical information processing apparatus provided in an aspect of the present embodiment includes an acquisition unit that acquires a plurality of first ultrasonic data within a predetermined time, an extraction unit that, at each position in a frame represented by the first ultrasonic data, extracts identification information that identifies a magnitude relation between a signal value corresponding to the position and a signal value corresponding to a surrounding area of the position, from each of the first ultrasonic data, and an output unit that outputs second ultrasonic data, based on at least one of the first ultrasonic data, or ultrasonic data obtained by performing a predetermined process on the first ultrasonic data, and the identification information extracted from each of the first ultrasonic data.

At each position in the frame, the extraction unit may extract information that identifies whether the signal value corresponding to the position is greater than the signal value corresponding to the surrounding area of the position, as the identification information.

The medical information processing apparatus includes a generation unit that generates filter information corresponding to each position in the frame, based on the identification information extracted from each of the first ultrasonic data by the extraction unit, wherein the output unit may output the second ultrasonic data, based on at least one of the first ultrasonic data, or ultrasonic data obtained by performing a predetermined process on the first ultrasonic data, and the filter information.

The extraction unit may assign weight information defined according to each of the first ultrasonic data to the identification information.

The surrounding area with which the magnitude relation between the signal values is identified by the extraction unit, is a position in a certain spatial direction and distance with respect to the position, and the extraction unit may assign weight information defined according to at least one of the direction and distance, to the identification information.

The acquisition unit may acquire addition data that is obtained by performing ultrasonic scanning on a subject, and that is obtained by performing addition processing of a plurality of frame data continuous in a temporal direction, as the first ultrasonic data.

At each position in the frame represented by the first ultrasonic data, the output unit may set ultrasonic data obtained by performing a process of extracting a signal value corresponding to the position, based on the magnitude relation between a signal value corresponding to the position and a signal value corresponding to a surrounding area of the position, as the ultrasonic data obtained by performing the predetermined process, and output the second ultrasonic data, based on the ultrasonic data and the identification information.

At each position in the frame represented by the first ultrasonic data, the output unit may set synthetic data in which ultrasonic data obtained by performing a process of extracting a signal value corresponding to the position, based on the magnitude relation between a signal value corresponding to the position and a signal value corresponding to a surrounding area of the position is synthesized with the first ultrasonic data, as the ultrasonic data obtained by performing the predetermined process, and output the second ultrasonic data, based on the ultrasonic data and the identification information.

The acquisition unit may acquire the first ultrasonic data obtained by performing ultrasonic scanning in the presence of a contrast agent.

The acquisition unit may acquire the first ultrasonic data obtained by performing ultrasonic scanning in the absence of a contrast agent.

An ultrasonic diagnostic apparatus provided in an aspect of the present embodiment includes an execution unit that causes an ultrasonic probe to perform ultrasonic scanning;

an acquisition unit that acquires a plurality of first ultrasonic data within a predetermined time, an extraction unit that, at each position in a frame represented by the first ultrasonic data, extracts identification information that identifies a magnitude relation between a signal value corresponding to the position and a signal value corresponding to a surrounding area of the position, from each of the first ultrasonic data, and an output unit that outputs second ultrasonic data, based on at least one of the first ultrasonic data, or ultrasonic data obtained by performing a predetermined process on the first ultrasonic data, and the identification information extracted from each of the first ultrasonic data.

A medical information processing method provided in an aspect of the present embodiment includes an acquisition step of acquiring a plurality of first ultrasonic data within a predetermined time, an extraction step of extracting identification information that identifies a magnitude relation between a signal value corresponding to each position in a frame represented by the first ultrasonic data and a signal value corresponding to a surrounding area of the position, from each of the first ultrasonic data, and an output step of outputting second ultrasonic data, based on at least one of the first ultrasonic data, or ultrasonic data obtained by performing a predetermined process on the first ultrasonic data, and the identification information extracted from each of the first ultrasonic data.

An ultrasonic diagnostic apparatus provided in an aspect of the present embodiment includes an ultrasonic probe that transmits an ultrasonic wave to a subject, and receives the ultrasonic wave reflected by the subject, an acquisition unit that acquires first ultrasonic data of the subject, based on the ultrasonic wave received by the ultrasonic probe, an extraction unit that extracts identification information that identifies a position of a local peak, for each predetermined region of a frame represented by the first ultrasonic data, and an output unit that outputs second ultrasonic data, based on the identification information extracted by the extraction unit, and ultrasonic data representing an object to be processed.

The extraction unit may extract the identification information, by identifying a position where a signal value is relatively high as the position of a local peak for each local region of the frame represented by the first ultrasonic data.

The acquisition unit may acquire a plurality of the first ultrasonic data within a predetermined time, the extraction unit may extract identification information that identifies a position of a local peak, for each frame represented by the first ultrasonic data, and the output unit may output the second ultrasonic data, based on the identification information extracted for each of the frames, and the ultrasonic data.

At each position in the frame, the extraction unit may extract information that identifies a magnitude relation between a signal value corresponding to the position and a signal value corresponding to a surrounding area of the position, as the identification information.

At each position in the frame, the extraction unit may extract information that identifies whether the signal value corresponding to the position is greater than the signal value corresponding to the surrounding area of the position, as the identification information.

The surrounding area may be a position in a spatial direction and range with respect to the position, or a position in a temporal direction and range with respect to the position.

In a predetermined range of the frame, the extraction unit may extract information that identifies a position having a higher signal value than that of a surrounding area, as the identification information.

The surrounding area may be a position in a spatial direction and range with respect to the position, or a position in a temporal direction and range with respect to the position.

At each position in the frame represented by the first ultrasonic data, the extraction unit may extract the identification information.

The ultrasonic data representing the object to be processed may be, at least one of the first ultrasonic data, or ultrasonic data obtained by performing a predetermined process on the first ultrasonic data.

The ultrasonic diagnostic apparatus includes a generation unit that generates filter information corresponding to each position in the frame, based on the identification information extracted from each of the first ultrasonic data by the extraction unit, wherein the output unit may output the second ultrasonic data, based on the filter information and ultrasonic data representing the object to be processed.

The extraction unit may assign weight information defined according to each of the first ultrasonic data to the identification information.

The surrounding area with which the magnitude relation between the signal values is identified by the extraction unit, is a position in a certain spatial direction and distance with respect to the position, or a position in a certain temporal direction and distance with respect to the position, and the extraction unit may assign weight information defined according to at least one of the direction and distance, to the identification information.

The acquisition unit may acquire addition data that is obtained by performing ultrasonic scanning on a subject, and that is obtained by performing addition processing of a plurality of frame data continuous in the temporal direction, as the first ultrasonic data.

At each position in the frame represented by the first ultrasonic data, the output unit may set ultrasonic data obtained by performing a process of extracting a signal value corresponding to the position, based on the magnitude relation between a signal value corresponding to the position and a signal value corresponding to a surrounding area of the position, as the ultrasonic data obtained by performing the predetermined process, and output the second ultrasonic data, based on the ultrasonic data and the identification information.

At each position in the frame represented by the first ultrasonic data, the output unit may set synthetic data in which ultrasonic data obtained by performing a process of extracting a signal value corresponding to the position, based on the magnitude relation between a signal value corresponding to the position and a signal value corresponding to a surrounding area of the position is synthesized with the first ultrasonic data, as the ultrasonic data obtained by performing the predetermined process, and output the second ultrasonic data, based on the ultrasonic data and the identification information.

The acquisition unit may acquire the first ultrasonic data obtained by performing ultrasonic scanning in the presence of a contrast agent.

The acquisition unit may acquire the first ultrasonic data obtained by performing ultrasonic scanning in the absence of a contrast agent.

At each position in the frame, the extraction unit may extract information that identifies the magnitude relation between a signal value corresponding to the position and a signal value corresponding to the surrounding area of the position, after multiplying a predetermined coefficient by one of the signal values, as the identification information.

At each position in the frame, the extraction unit may extract information that identifies variance between a signal value corresponding to the position and a signal value corresponding to a surrounding area of the position, as the identification information.

The acquisition unit may acquire three-dimensional ultrasonic data, for each of the first ultrasonic data within the predetermined time.

In a mode of extracting the identification information, the acquisition unit may acquire the first ultrasonic data collected by increasing line density.

The extraction unit may extract the identification information, by dividing the first ultrasonic data into a plurality of division data, generating extraction data in which the position of the local peak is extracted for each division data, and allocating each extraction data to a corresponding position on the first ultrasonic data.

A medical information processing apparatus provided in an aspect of the present embodiment includes an acquisition unit that acquires first ultrasonic data of a subject, an extraction unit that extracts identification information that identifies a position of a local peak, for each predetermined region of a frame represented by the first ultrasonic data, and an output unit that outputs second ultrasonic data, based on the identification information extracted by the extraction unit, and ultrasonic data representing an object to be processed.

A medical information processing method provided in an aspect of the present embodiment includes an acquisition step of acquiring first ultrasonic data of a subject, an extraction step of extracting identification information that identifies a position of a local peak, for each predetermined region of a frame represented by the first ultrasonic data, and an output step of outputting second ultrasonic data, based on the identification information extracted by the extraction step, and ultrasonic data representing an object to be processed.

### BRIEF DESCRIPTION OF THE DRAWINGS

FIG. 1 is a block diagram illustrating an example of a configuration of an ultrasonic diagnostic apparatus according to a first embodiment;
FIG. 2 is a flowchart illustrating a processing procedure of the ultrasonic diagnostic apparatus according to the first embodiment;
FIG. 3 is a diagram for explaining first ultrasonic data according to the first embodiment;
FIG. 4 is a diagram for explaining extraction of a signal component according to the first embodiment;
FIG. 5 is a diagram for explaining an example of extraction processing according to the first embodiment;
FIG. 6 is a diagram for explaining filter information according to the first embodiment;
FIG. 7A is a diagram for explaining an example of generating the filter information according to the first embodiment;
FIG. 7B is a diagram for explaining an example of generating the filter information according to the first embodiment;
FIG. 8 is a diagram for explaining second ultrasonic data according to the first embodiment;
FIG. 9A is a diagram illustrating ultrasonic data according to a comparative example;
FIG. 9B is a diagram illustrating second ultrasonic data obtained by performing a process according to the first embodiment;
FIG. 10 is a diagram for explaining an example of the process according to another embodiment;
FIG. 11A is a diagram for explaining an example of a process according to another embodiment;
FIG. 11B is a diagram for explaining an example of the process according to another embodiment;
FIG. 12 is a diagram for explaining an example of the process according to another embodiment;
FIG. 13 is a diagram for explaining an example of processing results of a process according to the comparative example; and
FIG. 14 is a diagram for explaining an example of processing results of the process according to another embodiment.

### DETAILED DESCRIPTION

Hereinafter, embodiments of an ultrasonic diagnostic apparatus, a medical information processing apparatus, and a medical information processing method according to the present application will be described in detail, with reference to the accompanying drawings. The ultrasonic diagnostic apparatus, the medical information processing apparatus, and the medical information processing method according to the present application are not limited to the following embodiments.

### First Embodiment

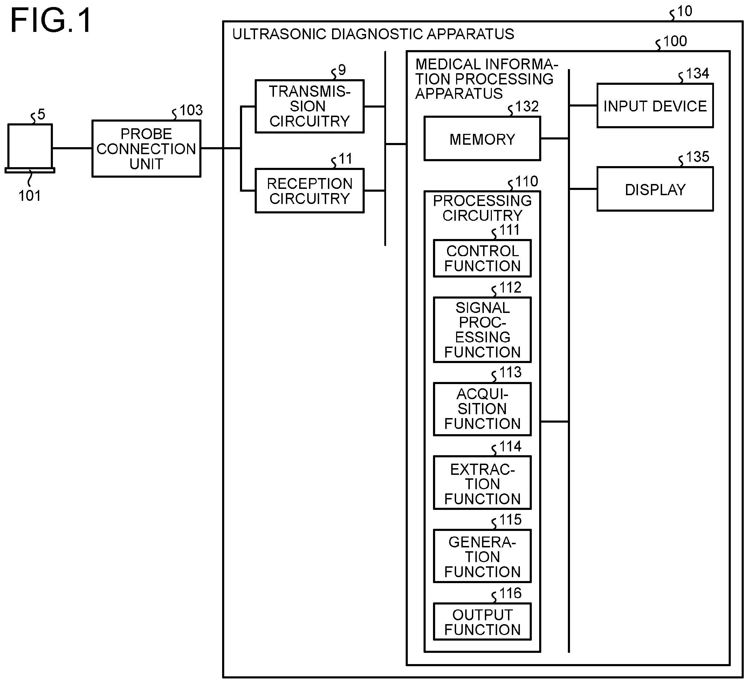

FIG. 1 is a block diagram illustrating an example of a configuration of an ultrasonic diagnostic apparatus 10 according to a first embodiment. The ultrasonic diagnostic apparatus 10 is an apparatus that generates ultrasonic data on the basis of a reception signal (reflected wave signal) received from an ultrasonic probe 5. The ultrasonic diagnostic apparatus 10 illustrated in FIG. 1 is an apparatus that can generate two-dimensional ultrasonic data, on the basis of a reception signal received from a one-dimensional ultrasonic probe 5 (ultrasonic probe in which transducer elements are arranged one-dimensionally), and that can generate three-dimensional ultrasonic data, on the basis of the reception signal received from a two-dimensional ultrasonic probe 5 (ultrasonic probe in which transducer elements are arranged two-dimensionally).

For example, the ultrasonic probe 5 is an electronic scanning-type probe, and the tip end of which includes a plurality of transducer elements 101 arranged one-dimensionally or two-dimensionally. Each of the transducer elements 101 is a piezoelectric element (electromechanical conversion element) that performs mutual conversion between electric signals (voltage pulse signals) and ultrasonic waves (acoustic waves). The ultrasonic probe 5 transmits ultrasonic waves to a subject from the transducer elements 101, and receives reflected ultrasonic waves from the subject by the transducer elements 101. The reflected acoustic waves reflect the difference in acoustic impedance in the subject. The reflected ultrasonic wave obtained when the transmitted ultrasonic pulse is reflected by the moving blood flow, the surface of the heart wall, or the like, depends on a velocity signal component of a moving body in the ultrasonic wave transmission direction, and undergoes a frequency shift due to the Doppler effect.

A probe connection unit 103 is connected to the ultrasonic probe 5, and transmits and receives ultrasonic waves to and from the ultrasonic probe 5. The probe connection unit 103 may be connected to the ultrasonic probe 5 in wired or wireless manner. In case of wired connection, the probe connection unit 103 includes a connector unit (receptacle) to connect a connector (plug) of the ultrasonic probe 5. In case of wireless connection, the probe connection unit 103 includes a communication unit for wirelessly communicating with the ultrasonic probe 5.

The ultrasonic diagnostic apparatus 10 includes transmission circuitry 9, reception circuitry 11, and a medical information processing apparatus 100.

The transmission circuitry 9 is a transmission unit that outputs a pulse signal (drive signal) to the transducer elements 101. By applying a pulse signal to the transducer elements 101 with a time difference, ultrasonic waves with different delay times are transmitted from the transducer elements 101. Hence, a transmission ultrasonic beam is formed. The direction and focus of the transmission ultrasonic beam can be controlled, by selectively changing the transducer element 101 to which the pulse signal is applied (that is, the transducer element 101 to be driven), or changing the delay time (application timing) of the pulse signal. By sequentially changing the direction and focus of the transmission ultrasonic beam, an observation region inside the subject is scanned. Moreover, the transmission ultrasonic beam that is a plane wave (focus is far away) or a diffuse wave (focus point is opposite to the ultrasonic wave transmission direction of the transducer elements 101) may be formed, by changing the delay time of the pulse signal. Alternatively, the transmission ultrasonic beam may be formed using one transducer element or some of the transducer elements 101. The transmission circuitry 9 generates a transmission ultrasonic wave having a predetermined transmission waveform in the transducer elements 101, by transmitting a pulse signal having a predetermined transmission waveform to the transducer elements 101.

The reception circuitry 11 is a reception unit that inputs an electric signal output from the transducer element 101 that has received the reflected ultrasonic wave, as a reception signal. The reception signal is input to a processing circuitry 110. In the present embodiment, an analog signal output from the transducer element 101, and digital data obtained by sampling (digitally converting) the analog signal, are both referred to as a reception signal without any particular differentiation.

The medical information processing apparatus 100 is connected to the transmission circuitry 9 and the reception circuitry 11, and processes the signal received from the reception circuitry 11, and controls the transmission circuitry 9. The medical information processing apparatus 100 includes the processing circuitry 110, a memory 132, an input device 134, and a display 135.

The memory 132 includes a semiconductor memory element such as a Random Access Memory (RAM) and a flash memory, a hard disk, an optical disc, and the like. The memory 132 is a memory that stores therein data such as display image data generated by the processing circuitry 110. Moreover, the memory 132 can also store therein the reception signal (reflected wave signal) output by the reception circuitry 11. In addition, according to the needs, the memory 132 stores therein a control program for performing ultrasonic wave transmission and reception, image processing, and display processing; diagnostic information (for example, patient's ID, doctor's opinion, and the like); and various data such as a diagnostic protocol and various body marks.

The input device 134 receives various instructions and information inputs from an operator. For example, the input device 134 includes a trackball, a switch button, a mouse, a keyboard, a touch pad that performs input operations by touching the operation surface, a touch monitor in which a display screen and a touch pad are integrated, a non-contact input circuit using an optical sensor, and an input interface device such as a voice input circuit. The input interface device is connected to the processing circuitry 110, which will be described later, converts the input operation received from the operator to an electric signal, and outputs the converted electric signal to the processing circuitry 110. In the present specification, the input interface device is not limited to that including a physical operation components such as a mouse and a keyboard. For example, an example of the input interface device also includes an electric signal processing circuit that receives an electric signal corresponding to the input operation from an external input device provided separately from the device, and that outputs the electric signal to the processing circuitry 110.

Under the control of the processing circuitry 110, the display 135 displays a Graphical User Interface (GUI) for receiving an input of imaging conditions, and various images. For example, the display 135 includes a display interface device such as a liquid crystal display.

By controlling each unit of the ultrasonic diagnostic apparatus 10, the processing circuitry 110 controls the entire ultrasonic diagnostic apparatus 10. For example, the processing circuitry 110 executes a computer program stored in the memory 132, and thus functions as a control function 111, a signal processing function 112, an acquisition function 113, an extraction function 114, a generation function 115, and an output function 116. In this example, the control function 111 is an example of an execution unit. Moreover, the acquisition function 113 is an example of an acquisition unit. Furthermore, the extraction function 114 is an example of an extraction unit. Still furthermore, the generation function 115 is an example of a generation unit. Still furthermore, the output function 116 is an example of an output unit. In FIG. 1, the functions are implemented by a single processing circuitry 110. However, the functions may also be implemented by combining multiple independent processors. Moreover, a specific function may be configured by a dedicated independent circuit such as an Application Specific Integrated Circuit (ASIC).

Furthermore, for example, the term "processor" used in the above description refers to a Central Processing Unit (CPU) and a Graphical Processing Unit (GPU), or a circuit such as an Application Specific Integrated Circuit (ASIC) and a programmable logic device (for example, a Simple Programmable Logic Device (SPLD), a Complex Programmable Logic Device (CPLD), and a Field Programmable Gate Array (FPGA)). The processor implements the functions by reading and executing the computer programs stored in the memory 132.

The ultrasonic diagnostic apparatus 10 configured as described above performs processes roughly classified as follows.

### Process 1

A process of acquiring addition data that is obtained by performing ultrasonic scanning on a subject, and that is obtained by performing addition processing of a plurality of frame data continuous in the temporal direction, as first ultrasonic data.

### Process 2

At each position in the frame (image) represented by the first ultrasonic data, a process of extracting identification information that identifies the magnitude relation between a signal value corresponding to the position and a signal value corresponding to a surrounding area of the position, from each of a plurality of the first ultrasonic data.

### Process 3

At each position in the frame (image), a process of generating filter information corresponding to the position, on the basis of the identification information extracted from each of the first ultrasonic data.

### Process 4

A process of outputting second ultrasonic data, on the basis of at least one of the first ultrasonic data (for example, the latest first ultrasonic data) and the filter information.

The ultrasonic diagnostic apparatus 10 that performs Process 1 to Process 4 improves the image quality of an ultrasonic image (particularly, an ultrasonic image including fine flow paths and fluid), by a high time resolution and a low computational load.

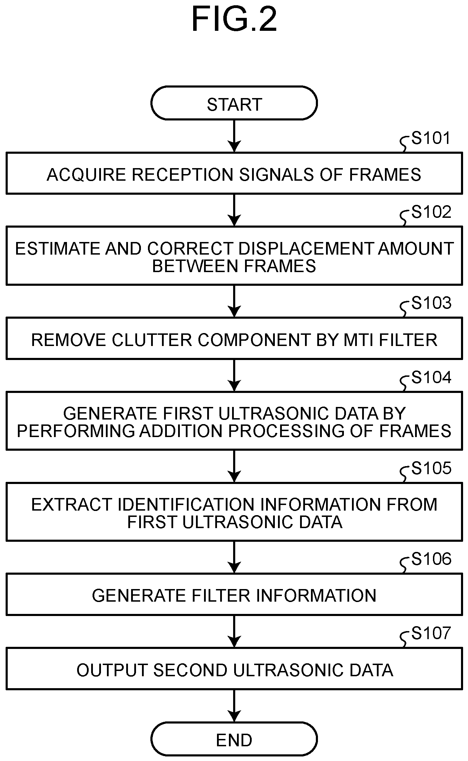

Hereinafter, an operation of the ultrasonic diagnostic apparatus 10 will be described in detail with reference to FIG. 2.

The control function 111 controls the transmission circuitry 9 and the reception circuitry 11, causes the ultrasonic probe 5 to perform ultrasonic scanning, and acquires reception signals (for example, CH data) of a plurality of frames (step S101). Specifically, the control function 111 collects a plurality of frame data continuous in the temporal direction (a plurality of frame data within a predetermined time) that are obtained by performing ultrasonic scanning, at a predetermined frame rate. The frame data includes an amplitude value (signal value) of a signal corresponding to each pixel that makes up a single image (frame).

The signal processing function 112 performs phase addition and quadrature detection processing on the collected reception signals. The phase addition processing is a process of adding the reception signals of the transducer elements 101 by changing the delay time and weight of each transducer element 101, and is also referred to as Delay and Sum (DAS) beamforming. The quadrature detection processing is a process of converting a reception signal into a baseband in-phase signal and orthogonal signal, and acquiring IQ data and measurement data that represents the absolute value of the IQ data or the like. In addition, adaptive beamforming, model-based processing, processing using machine learning, and the like may be performed on the reception signal.

Moreover, the signal processing function 112 estimates the displacement amount of the tissue due to the body movement or the like of the subject between the frame data, and corrects each frame data on the basis of the estimated results (step S102). Specifically, the signal processing function 112 calculates the displacement amount of the tissue due to the body movement or the like between the frames from the frame data, and corrects the frame data on the basis of the calculated displacement amount.

Moreover, the signal processing function 112 performs envelope detection processing, logarithmic compression processing, or the like, and generates B-mode data in which the signal intensity at each point in the observation region is represented with luminance. Furthermore, the signal processing function 112 generates blood flow data obtained by extracting the information derived from blood flow from the measurement data. For example, the signal processing function 112 applies the Moving Target Indicator (MTI) filter to the frame data (step S103). Consequently, information derived from stationary tissue or information derived from slow-moving tissue (tissue signal component (clutter)) is reduced from the frames, and information derived from blood flow (blood flow signal component) is extracted. As the MTI filter, a filter with fixed filter information, such as a Butterworth Infinite Impulse Response (IIR) filter or a Polynomial Regression Filter may also be used. The MTI filter may be an adaptive filter that changes the coefficients according to the input signal using eigenvalue decomposition, singular value decomposition, or the like.

Moreover, the signal processing function 112 can extract the information derived from blood flow, by decomposing the frame data into a plurality of bases by the eigenvalue decomposition, singular value decomposition, or the like, and removing the information derived from tissue by taking out a specific base. Furthermore, the signal processing function 112 can obtain a blood flow vector representing the size and direction of blood flow, by obtaining a velocity vector of each set of coordinates in the reception signal data, by using a method such as a vector Doppler method, a speckle tracking method, and a Vector Flow Mapping method. In addition to the methods illustrated in this example, a method of extracting the information derived from blood flow included in frame data (measurement data), or a method of removing the information derived from tissue may also be adopted.

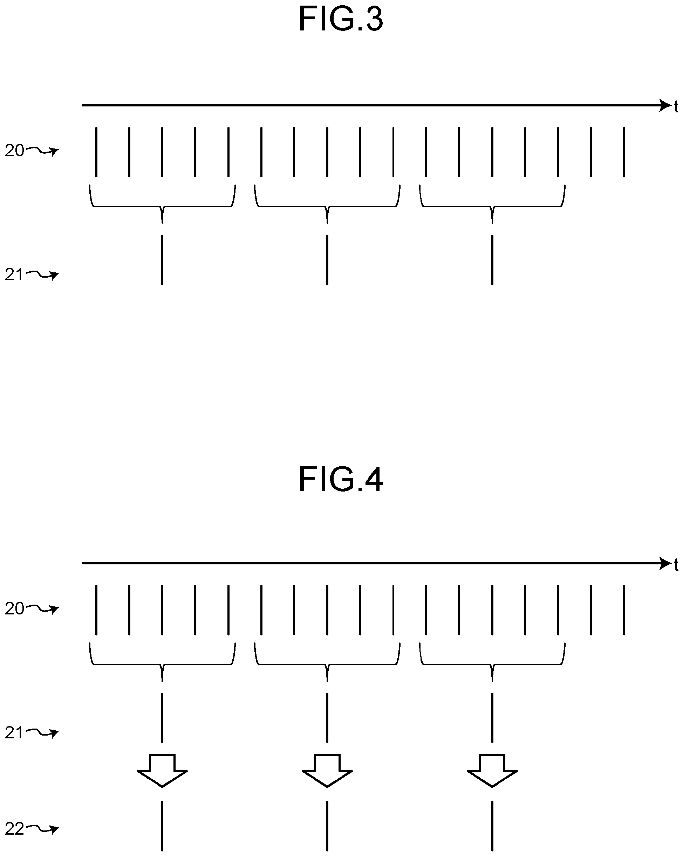

The acquisition function 113 acquires the first ultrasonic data within a predetermined time, on the basis of the results obtained by performing ultrasonic scanning on the subject. For example, the acquisition function 113 acquires frame data that are obtained by performing ultrasonic scanning on the subject and that are continuous in the temporal direction, and performs addition processing of the acquired frame data (step S104). For example, as illustrated in FIG. 3, the acquisition function 113 performs addition processing of the frame data 20 that are continuous in the temporal direction, and from which the clutter component is removed by the process at step S103, for every fixed number of frames. For example, the fixed number of frames (number of packets) in this process is about several tens to hundreds. The acquisition function 113 acquires the addition data obtained by the addition processing, as first ultrasonic data 21.

The Signal-Noise Ratio (SN ratio) of the first ultrasonic data 21 is higher than that of each frame data 20 before being added. For example, the first ultrasonic data 21 (addition data) is blood flow data (power Doppler data) in which the information derived from blood flow is emphasized. Moreover, the first ultrasonic data 21 represents the boundary of a flow path (for example, the shape of blood vessel), obtained by averaging the signals of the fluid from which clutter is removed, within a predetermined time. The frame represented by the first ultrasonic data 21 includes a signal value (amplitude value) representing a speckle pattern generated by constructive interference and destructive interference between the ultrasonic waves reflected from the fluid, and a signal value representing other noise (artifact and the like) that is generated instantaneously or intermittently on the time axis.

As illustrated in FIG. 4, the extraction function 114 extracts identification information 22 from each of the first ultrasonic data 21 (step S105). The identification information is information that, at each position in the frame (image) represented by the first ultrasonic data 21, identifies the magnitude relation between the signal value corresponding to the position and the signal value corresponding to the surrounding area of the position.

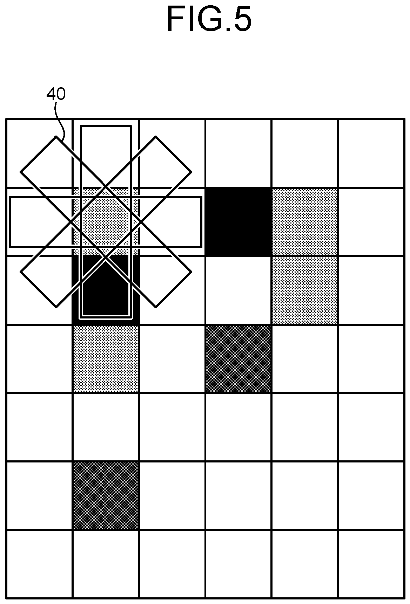

As illustrated in FIG. 5, the extraction function 114 disposes a kernel 40 at the attention position in the frame (image) represented by the first ultrasonic data 21, and compares the magnitude relation between the signal value corresponding to the attention position and the signal value corresponding to the position of an image within a range (surrounding area of the attention position) where the kernel 40 is disposed. For example, at each position in the frame, the extraction function 114 acquires information that identifies whether the signal value corresponding to the position is greater than the signal value corresponding to the surrounding area of the position, as the identification information. That is, the identification information can also be referred to as information that, for each local region in the frame, identifies the position (local peak position) where the signal value is relatively high.

Specifically, when a kernel with the aspect ratio of 3 in height × 1 in width is used, the extraction function 114 disposes the center (second square) of the kernel 40 at the attention position, and identifies whether the signal value corresponding to the position is greater than the signal value corresponding to the positions at both ends (first square and third square) of the kernel 40. If the signal value corresponding to the attention position is greater than the signal value corresponding to the surrounding area of the position, the extraction function 114 represents the attention position with "1", for example. On the other hand, if the signal value corresponding to the attention position is smaller than the signal value corresponding to the surrounding area of the position, the extraction function 114 represents the attention position by "0", for example. Then, the extraction function 114 extracts the identification information corresponding to the frame (image) represented by the first ultrasonic data 21, by performing this process at each position in the frame.

That is to say, with this extraction processing, the first ultrasonic data 21 is converted into a binary image in which each pixel of the frame (image) is represented by "0" or "1", and the amplitude value (signal value) of the signal corresponding to each pixel that makes up the first ultrasonic data 21 will be lost.

In the example of FIG. 5, at each position in the frame represented by the first ultrasonic data, the identification information is extracted by disposing the kernel 40 in four directions of the vertical, horizontal, diagonal upper right, and diagonal upper left directions. However, the shape and size of the kernel is not limited thereto. Moreover, in the example of FIG. 5, an object is extracted in pixel units. However, the identification information at each position in the frame may also be extracted in group units including multiple pixels. When the identification information is extracted in group units including multiple pixels, the average value or the integrated value of the signal values at each of the positions that make up the group, may be used as the signal value corresponding to each position in the frame.

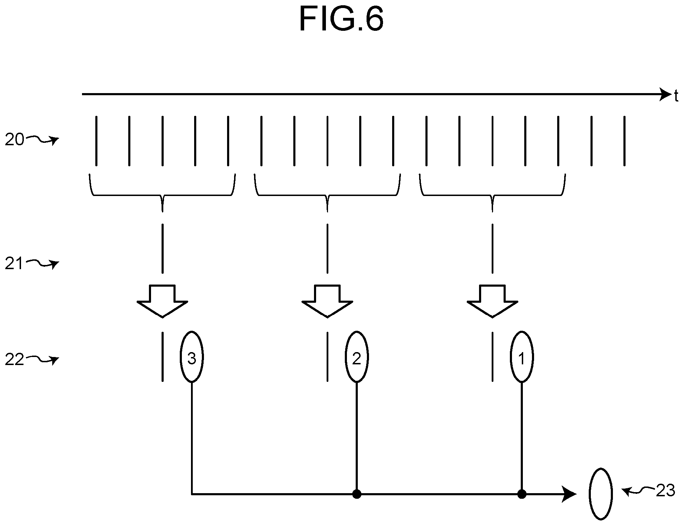

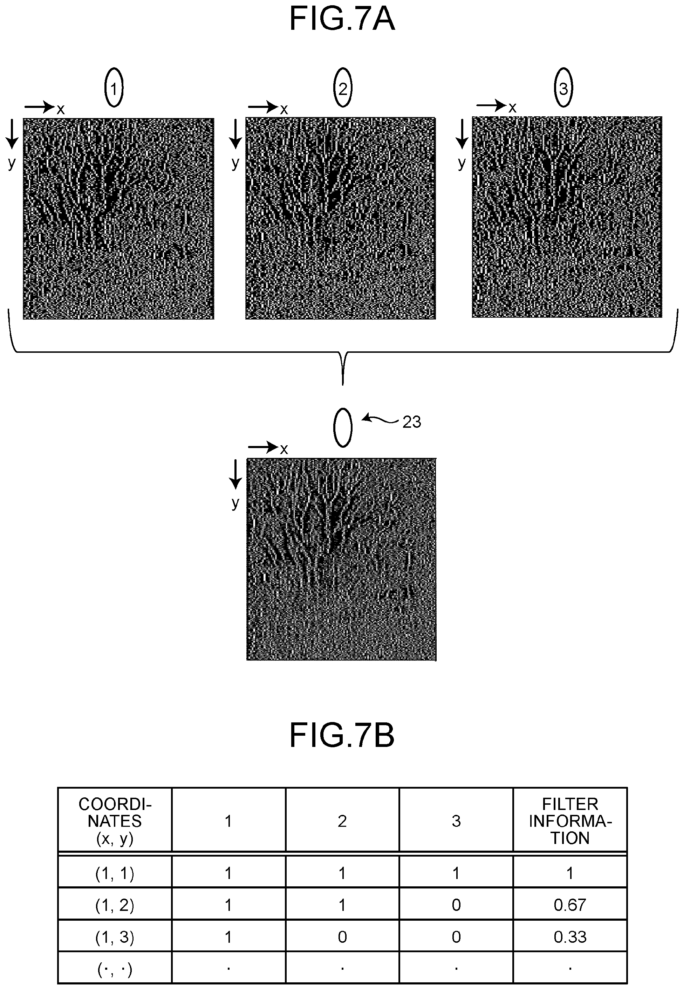

The generation function 115 generates filter information corresponding to each position in the frame, on the basis of the identification information 22 extracted from each of the first ultrasonic data 21, by the extraction function 114 (step S106). For example, as illustrated in FIG. 6 and FIG. 7A, the generation function 115 generates filter information 23 using the identification information 22 of three frames (1 to 3 illustrated in FIG. 6). For example, as illustrated in FIG. 7B, the filter information 23 is obtained according to the value represented by each piece of the identification information 22 at the positions (coordinates (x, y)) corresponding to each other between the frames. For example, if all three frames are "1" at the positions corresponding to each other in the identification information 22, the filter information at the position will be "1". Moreover, if one of the three frames is "1" and two of the three frames is "0" at the positions corresponding to each other, the filter information at the position will be "0.33". If all three frames are "0" at the positions corresponding to each other, the filter information at the position will be "0".

In this manner, of a plurality of pieces of the identification information 22 within a predetermined time, a larger value is set to the filter information 23, with an increase in the number of times the frame becomes "1" at the positions corresponding to each other. That is, with an increase in the number of times the frame becomes "1" at the positions corresponding to each other in the identification information 22, the signal values greater than that of the surrounding area is continuously obtained at the position, and there is a high possibility of the presence of fluid. Hence, at such a position, weight coefficient as filter information is set to a high value ("1"). On the other hand, if "0" is included in the positions corresponding to each other in the identification information 22, there is a high possibility that there is noise (artifact and the like) generated instantaneously or intermittently on the time axis. Hence, at such a position, as the number of zeros increases, the weight coefficient as filter information is set to a low value ("0.67", "0.33", or "0").

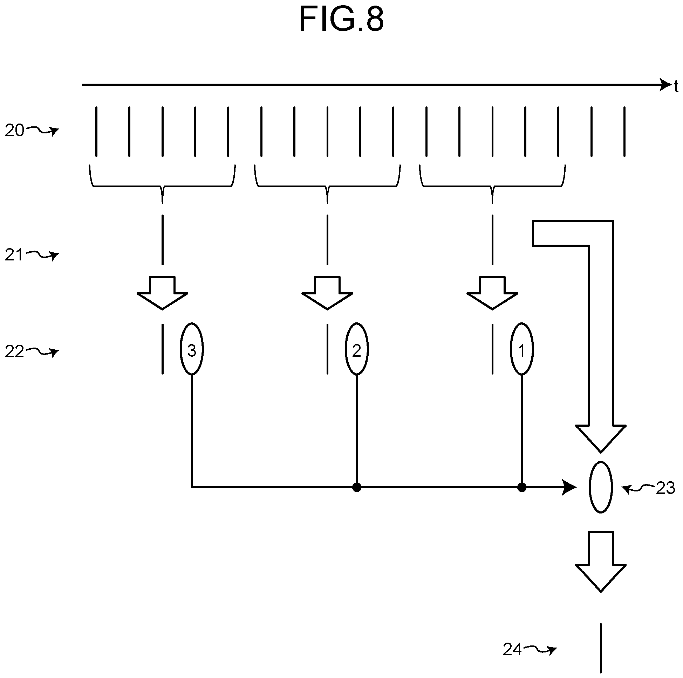

The output function 116 outputs second ultrasonic data, on the basis of the filter information 23 generated by the generation function 115, and at least one of the first ultrasonic data 21 (S107). For example, as illustrated in FIG. 8, the output function 116 outputs second ultrasonic data 24, on the basis of the latest first ultrasonic data 21 among the first ultrasonic data 21, and the filter information 23.

Specifically, the output function 116 generates and outputs the second ultrasonic data 24, by multiplying the signal value (amplitude value) representing each position in the frame represented by the latest first ultrasonic data 21, by the filter information (weight coefficient) set for each corresponding position in the filter information 23. In the second ultrasonic data 24, with the filter information 23, a position where there is a high possibility of fluid in the frame represented by the first ultrasonic data 21 is multiplied by a weight coefficient with a high value. Hence, the position of fluid will be emphasized. On the other hand, in the second ultrasonic data 24, with the filter information 23, a position where there is a high possibility of noise (artifact and the like) generated instantaneously or intermittently in the frame represented by the first ultrasonic data 21 is multiplied by a weight coefficient with a small value. Hence, the noise will be reduced.

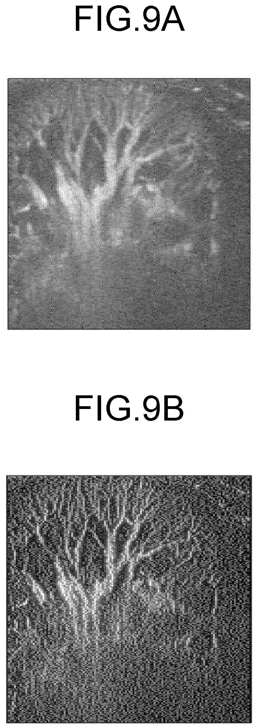

By referring to FIG. 9A and FIG. 9B, the ultrasonic image (blood flow image) represented by the second ultrasonic data 24 will be compared with a conventional ultrasonic image. FIG. 9A is a diagram illustrating a conventional ultrasonic image. FIG. 9B is a diagram illustrating an ultrasonic image represented by the second ultrasonic data 24. The ultrasonic image illustrated in FIG. 9A is obtained by averaging the signals of fluid from which clutter is removed within a predetermined time. The image of the flow path cannot be displayed at high quality with an increase in the fineness of the flow path. On the other hand, in the ultrasonic image illustrated in FIG. 9B, the image of running of fine blood vessels can be displayed at high quality.

As described above, with the ultrasonic diagnostic apparatus 10 according to the first embodiment, it is possible to improve the image quality of an ultrasonic image, by a high time resolution and a low computational load.

Moreover, according to the first embodiment, the acquisition function 113 acquires the addition data that is obtained by performing ultrasonic scanning on the subject, and that is obtained by performing addition processing of the frame data continuous in the temporal direction, as the first ultrasonic data 21. Consequently, it is possible to reduce the number of frames from which the identification information 22 is extracted, and use the ultrasonic data with a high SN ratio.

In the first embodiment, the output function 116 outputs the second ultrasonic data 24 on the basis of the latest first ultrasonic data 21 among the ultrasonic data 21 and the filter information 23. However, it is not limited thereto, and the output function 116 may also output the second ultrasonic data 24 on the basis of the other first ultrasonic data 21 that is not the latest first ultrasonic data 21 among the first ultrasonic data 21, and the filter information 23.

Moreover, the output function 116 may perform the same addition processing as that at the step S104 on the first ultrasonic data 21, and output the second ultrasonic data 24 on the basis of the data obtained by the addition processing and the filter information 23. In this case, by multiplying the data in which the SN ratio is higher than that of the first ultrasonic data 21 by the filter information 23, the effects of further improving the image quality of the ultrasonic image can be expected, than that described in the embodiment.

Furthermore, in the first embodiment, the output function 116 outputs the second ultrasonic data 24 on the basis of the filter information 23 generated by the generation function 115, and the first ultrasonic data 21. However, it is not limited thereto. For example, the output function 116 may also output the second ultrasonic data 24, on the basis of the identification information 22 extracted from each of the first ultrasonic data 21 by the extraction function 114, and at least one of the first ultrasonic data 21, or ultrasonic data obtained by performing a predetermined process on the first ultrasonic data 21.

In this case, the output function 116 may also use a trained model that has been trained in advance. The output function 116 may input the identification information 22 extracted from each of the first ultrasonic data 21, and at least one of the first ultrasonic data 21, or ultrasonic data obtained by performing a predetermined process on the first ultrasonic data 21, to the trained model, and obtain the data output by the trained model, as the second ultrasonic data 24.

Still furthermore, in extracting the identification information at step S105, the extraction function 114 may also perform extraction processing that extracts a signal value with a relatively high signal value (signal value having a local peak) for each local region in the frame (at each position in the frame represented by the first ultrasonic data 21, a process of extracting a signal value corresponding to the position on the basis of the magnitude relation between the signal value corresponding to the position and the signal value corresponding to the surrounding area of the position). In this case, the output function 116 may also set ultrasonic data obtained as the results of the extraction processing performed by the extraction function 114, as the ultrasonic data obtained by performing a predetermined process, and output the second ultrasonic data 24, on the basis of the ultrasonic data and the filter information 23.

Moreover, the output function 116 may also set synthetic data in which the ultrasonic data obtained as the results of the extraction processing performed by the extraction function 114 is synthesized with the latest first ultrasonic data 21, as the ultrasonic data obtained by performing a predetermined process, and output the second ultrasonic data 24, on the basis of the ultrasonic data and the filter information 23.

Still furthermore, in the first embodiment, the identification information 22 is binarized by "0" or "1" with respect to each position in the frame represented by the first ultrasonic data 21. However, a list of positions where the signal value is higher than the signal value corresponding to the surrounding area may also be extracted as the identification information.

Still furthermore, weight information defined according to each of the first ultrasonic data may be assigned to the identification information extracted from each of the first ultrasonic data by the extraction function 114. For example, weight information the value of which is high with a decrease in the elapsed time of the first ultrasonic data 21 that is defined according to each of the first ultrasonic data may also be assigned to the identification information extracted from the first ultrasonic data. Consequently, weight according to the elapsed time may be applied to the identification information extracted from each of the first ultrasonic data.

Still furthermore, in the first embodiment, at each position in the frame, the extraction function 114 extracts the identification information that identifies the magnitude relation between the signal value corresponding to the position, and the signal value corresponding to the surrounding position in each direction and distance, by using the kernel 40 with the aspect ratio of 3 in height × 1 in width arranged in four directions of the vertical, horizontal, diagonal upper right, and diagonal upper left directions. However, it is not limited thereto. The surrounding area in which the magnitude relation between the signal values is identified by the extraction function 114 may be any position in a predetermined direction and distance with respect to each position in the frame represented by the first ultrasonic data 21. Moreover, the extraction function 114 may also assign the weight information defined according to at least one of the direction and distance to the identification information. Consequently, it is possible to apply the weight defined according to at least one of the direction and distance of the signal value to be compared, to the identification information extracted from each of the first ultrasonic data. The kernel 40 is optional as long the kernel 40 can define the extraction range of the identification information, and the shape, size, or the like may be set as appropriate.

Still furthermore, in the first embodiment, the addition data obtained by adding the frame data is acquired as the first ultrasonic data. However, the embodiment is not limited thereto, and each of the frame data may also be acquired as the first ultrasonic data. In other words, the acquisition function 113 may acquire each of the frames continuous in the temporal direction obtained by performing ultrasonic scanning as the first ultrasonic data. For example, the acquisition function 113 may set each of the five frame data 20 illustrated in FIG. 3 as the first ultrasonic data. In this case, the extraction function 114 may extract the identification information from each of the five frame data 20. Moreover, the generation function 115 may generate the filter information corresponding to each position in the frame, on the basis of the identification information extracted from each of the five frame data 20. Furthermore, the output function 116 may output the second ultrasonic data, on the basis of at least one of the five frame data 20 (for example, the latest frame data), or ultrasonic data obtained by performing a predetermined process on the frame data 20, and the filter information generated by the generation function 115.

In the first embodiment described above, the signal values between the pixels are compared in a single first ultrasonic data. However, the embodiment is not limited thereto. For example, the signal values may be compared between the first ultrasonic data that are temporally continuous. In such a case, the extraction function 114 may compare the signal values at the same coordinates, between the first ultrasonic data that are temporally continuous.

Moreover, in the embodiment described above, the acquisition function 113 acquires single addition data in which the SN ratio is improved by performing addition processing of the frame data, as the first ultrasonic data. However, the acquisition function 113 may also acquire the ultrasonic data in which the SN ratio is improved, without performing the addition processing. In such a case, for example, the acquisition function 113 may input the frame data 20 continuous in the temporal direction that is obtained by performing ultrasonic scanning on the subject, to the trained model that outputs single ultrasonic data in which the SN ratio is higher than that of the frame data, by inputting the frame data continuous in the temporal direction obtained by performing ultrasonic scanning, and acquire the single ultrasonic data output from the trained model as the first ultrasonic data 21. This trained model is trained by a dataset that uses frame data, as input data, and that uses single ultrasonic data in which the SN ratio is higher than that of each frame data, as teacher data, or the like. The acquisition function 113 inputs the frame data 20 into the trained model, and acquires the single ultrasonic data that is output from the trained model, and in which the SN ratio is higher than that of the frame data, as the first ultrasonic data 21.

Moreover, the acquisition function 113 may not perform the process of improving the SN ratio, and may acquire the first ultrasonic data 21 obtained on the basis of the results of ultrasonic scanning performed on the subject. For example, the acquisition function 113 may acquire the ultrasonic data in which the SN ratio received from an external workstation and the like is high, as the first ultrasonic data 21.

Moreover, the first embodiment described above is applied to the ultrasonic diagnostic apparatus. However, the embodiment is not limited thereto, and may also be applied to a medical information processing apparatus in addition to the ultrasonic diagnostic apparatus. For example, the first embodiment may also be applied to a medical information processing apparatus such as a workstation and a server that acquires the ultrasonic data obtained on the basis of the results of ultrasonic scanning performed on the subject. For example, the process described above may also be performed by the medical information processing apparatus such as a workstation and server, using the frame data collected in the past.

In addition, the fluid represented by the ultrasonic image in the above description is blood flow. Hence, the acquisition function 113 acquires the first ultrasonic data obtained by performing ultrasonic scanning in the absence of a contrast agent. However, the fluid may also be a contrast agent. In this case, the acquisition function 113 may acquire the first ultrasonic data obtained by performing ultrasonic scanning in the presence of a contrast agent.

Each component of each apparatus illustrated in the drawings for the description of the above embodiment is functionally conceptual, and does not necessarily have to be physically configured as illustrated. In other words, the specific form of distribution and integration of the devices is not limited to the illustrated one, and all or some of them can be configured in a functionally or physically distributed or integrated manner in any units according to various types of loads, use conditions, or the like. Furthermore, all or some of the processing functions performed by each device can be implemented by a CPU or a computer program analyzed and executed by the CPU, or can be implemented as hardware using wired logic.

Moreover, the methods described in the above embodiments can also be implemented by executing a prepared computer program on a computer such as a personal computer or a workstation. This computer program can be distributed via a network such as the Internet. Furthermore, this computer program can be recorded on a non-transitory computer-readable recording medium such as a hard disk, a flexible disk (FD), a CD-ROM, an MO, a DVD, a USB memory, and a Flash memory including an SD card memory, and executed by being read out from the non-transitory recording medium by a computer.

As described above, according to the embodiment, it is possible to obtain ultrasonic data with high visibility and high resolution.

While some embodiments have been described, these embodiments have been presented as examples, and are not intended to limit the scope of the invention. These embodiments may be implemented in various other forms, and various omissions, substitutions, modifications, and combinations of embodiments may be made without departing from the gist of the invention. These embodiments and their modifications are included in the scope and gist of the invention, and are also included in the invention described in the claims and the equivalents thereof.

### Other Embodiments

In the embodiment described above, the shape and size of the kernel used for extracting the local peak position is optional. For example, in addition to the kernel 40 with the aspect ratio of 3 in height × 1 in width illustrated in FIG. 5, kernels with the aspect ratio of 4 × 1, 5 × 1, 3 × 2, 3 × 3, or the like may also be used. In this example, the kernels described above may be used differently depending on the SN ratio of the data that is an object to be extracted from the local peak. For example, if an object to be extracted is the first ultrasonic data with a high SN ratio obtained by performing addition processing of the frame data, a kernel with a large aspect ratio (such as 3 × 1, 4 × 1, 5 × 1, or the like) may be used. This is because, in an image with a high SN ratio, the point noise and blood vessel structure are often discretely but clearly displayed. Hence, it is possible to efficiently suppress the point noise that appears in an isolated manner, by extending the observation range. On the other hand, if the frame data on which addition processing is not performed, and in which the SN ratio is not high is an object to be to extracted from the local peak, a kernel with a small aspect ratio (such as 2 × 1, 3 × 2, 3 × 3, or the like) may be used. Moreover, when obtaining the first ultrasonic data, the SN ratio is improved with an increase in the number of frame data to be added. Therefore, even in extracting the first ultrasonic data, the number of frame data to be added may be associated with the kernel to be used such that the kernel with a large aspect ratio in the frame is used with an increase in the number of frame data to be added.

Still furthermore, in the embodiment described above, the local peak position is extracted using one type of kernel. However, the embodiment is not limited thereto, and the local peak position may also be extracted by combining and using the kernels of two or more types. For example, the extraction function 114 extracts the position where the signal value is relatively high in the frame using the kernel of 5 × 1, and further extracts the position where the signal value is relatively high using the kernel of 3 × 3 with respect to the same frame. In this example, the extraction function 114 can set the position extracted by one of the kernel of 5 × 1 and the kernel of 3 × 3 as the local peak position, or set the position extracted by both of the kernel of 5 × 1 and the kernel of 3 × 3 as the local peak position. In other words, the extraction function 114 may extract the local peak position by combining the kernels of two or more types. As described above, the kernels of two or more types may also be combined depending on the SN ratio of the data that is an object to be extracted from the local peak. For example, if an object to be extracted is the first ultrasonic data with a high SN ratio obtained by performing addition processing of the frame data, a plurality of types of kernels with a large aspect ratio (kernels of 3 × 1, 4 × 1, or 5 × 1) may be combined, and the position extracted by one type of kernel may be set as the local peak position. On the other hand, if the frame data on which addition processing is not performed, and in which the SN ratio is not high is an object to be extracted from the local peak, a plurality of types of kernels with a small aspect ratio (kernels of 2 × 1, 3 × 2, or 3 × 3) may be combined, and the position extracted by all the types of kernels may be set as the local peak position.

Still furthermore, in the embodiment described above, in extracting the local peak position using a kernel, if the signal value of the attention position is greater than or the same as the signal value of the surrounding area of the attention position, the attention position is represented by "1". However, the embodiment is not limited thereto. For example, the signal values may also be compared after multiplying a predetermined coefficient by one of the signal values of the attention position or the signal value of the surrounding area. To give an example, the extraction function 114 may also compare the signal value of the attention position with the signal value of the surrounding position, after increasing the signal value of the surrounding position by X times (for example, 1.5 times or 0.9 times). Moreover, for example, if the signal value of the attention position is greater than the average value of the signal values at the surrounding positions, the attention position may be represented by "1". Furthermore, for example, the local peak position may be extracted by using the variance (standard deviation) between the signal values of the positions in the kernel. To give an example, if the signal value of the attention position is greater than or the same as the signal value of the surrounding area, and if the variance between the signal values of the positions (attention position and surrounding position) in the kernel is equal to or less than a threshold, the extraction function 114 may represent the attention position by "1". In this case, even if the signal value of the attention position is greater than or the same as the signal value of the surrounding area, if the variance exceeds the threshold, the extraction function 114 may represent the attention position by "0". This is because, in a region where point noise are scattered, the luminance values of the point noise are higher than those of the peripheral pixels. On the other hand, the variance value is also increased, because there is a large variation between the peripheral pixels and the point noise. When the dispersion value evaluation is combined with the comparison with peripheral pixel values, it is possible to efficiently suppress the point noise and extract the local peak.

Moreover, in the embodiment described above, the first ultrasonic data with three frames are used. However, the number of frames may be less than three (for example, two frames), or may be more than three (for example, five frames). Furthermore, a single frame may also be used. For example, the acquisition function 113 may acquire the first ultrasonic data of the subject, and the extraction function 114 may extract the identification information that identifies the local peak position for each predetermined region of one frame represented by the first ultrasonic data. In this case, the output function 116 may output the second ultrasonic data, on the basis of the identification information extracted from one frame by the extraction function 114, and the ultrasonic data representing an object to be processed. Still furthermore, the extraction function 114 may identify whether the attention position is the local peak position, by comparing each of the spatial directions (for example, four directions of the vertical, horizontal, diagonal upper right, and diagonal upper left directions) of each attention position in one frame represented by the first ultrasonic data, with another position within a predetermined range. In this case, the generation function 115 may set a weight coefficient as the filter information 23 at each attention position in one frame represented by the first ultrasonic data, according to the number of times the attention position is identified as the local peak for each direction by the extraction function 114. For example, when two directions of vertical and horizontal directions of the attention position in one frame represented by the first ultrasonic data are identified as the local peaks, the generation function 115 sets a weight coefficient of "0.5" for the attention position. When four directions (all directions) of vertical, horizontal, diagonal upper right, and diagonal upper left directions are identified as the local peaks, the generation function 115 sets a weight coefficient of "1" for the attention position. Then, the output function 116 may output the second ultrasonic data, on the basis of the filter information 23 generated by the generation function 115, and the ultrasonic data representing an object to be processed. The number of frames used for the first ultrasonic data may be changed by the user directly specifying the number of frames. Alternatively, the number of frames may be associated with the imaging target, examination type, and imaging mode specified by the user in advance, and the number of frames may be defined in conjunction with the user specifying the imaging target, examination type, and imaging mode. For example, an imaging area at a position away from the cardiovascular organs such as the finger joint is less likely affected by the body movement, and the imaging target seldom blurs during a long imaging time. Therefore, in the case of such an imaging target, the SN of the first ultrasonic data can be improved while increasing the number of frames to be added, by specifying a large number of frames such as five or ten. On the other hand, in an image of the abdominal area such as the liver, an object moves with the imaging time, as the object is slightly affected by the movement of the cardiovascular organs. Therefore, in the case of such an imaging target, it is possible to prevent the image quality of the first ultrasonic data from lowering that is caused by blurring of the imaging target, by specifying a small number of frames such as three. Alternatively, the total number of pixels on each frame may be counted for each frame, and if the number of counts changes significantly between the frames, it is possible to determine that blurring has occurred in the imaging target and the ultrasonic probe 5, and reduce the number of frames to be added to the minimum number (for example, three). On the other hand, if the total number of pixels on each frame does not change or exceed a predetermined threshold range over a predetermined time period, a process of increasing the number of frames to be added may be performed. Consequently, it is possible to increase the number of frames to be added in the time zone during which the imaging target is searched, and improve the SN ratio of the first ultrasonic data.

Still furthermore, in the embodiment described above, in extracting a local peak position, the signal values may also be compared between the first ultrasonic data that are temporally continuous, in addition to comparing the attention position with the other position in a certain spatial direction and range, at the same temporal position. In other words, the attention position may be compared with the other position in a certain temporal direction and range, at the same spatial position.

In such a case, the kernel disposed for the first ultrasonic data is disposed to include the time point to be paid attention in the temporal direction and the time point of the surrounding area, at the same spatial position. For example, when the signal values at the same coordinates are to be compared among the first ultrasonic data of three frames that are temporally continuous (first frame, second frame, and third frame), the signal values may be compared by setting the second frame as the time point to be paid attention and the first frame and the third frame as the time points of the surrounding areas. In this case, if the signal value of the first coordinates in the second frame is greater than or the same as the signal values of the first coordinates in the first frame and the third frame, the extraction function 114 extracts the first coordinates of the second frame as the local peak position. In extracting the local peak position as described above, the coordinates compared at the time point to be paid attention may also be extracted as the local peak position, if the variance between the signal values at the time points in the kernel is equal to or less than a threshold, in addition to the magnitude relation between the signal values. Moreover, the same coordinates described above may be those of a single pixel or multiple pixels. For example, when comparing the signal values of multiple pixels, the average values of the signal values of the multiple pixels in the frames may be compared.

For example, the extraction of local peak position in the first ultrasonic data that are temporally continuous may also be performed, by using a trained model that outputs the local peak position according to the input of the first ultrasonic data that are continuous in the temporal direction, in addition to the method using the kernel as described above. In such a case, the trained model described above is trained by a dataset that uses the first ultrasonic data that are temporally continuous, as input data, and that uses the local peak position extracted from the target data among the first ultrasonic data, as the teacher data, or the like. By inputting the first ultrasonic data that are temporally continuous to the trained model described above, the extraction function 114 extracts the local peak position in the target data. The process described above (process of extracting the local peak position using the data that are temporally continuous) may be performed on the first ultrasonic data that are temporally continuous as described above. However, the process may also be performed on the frame data that are temporally continuous.

Moreover, in extracting the identification information by extracting the local peak position in the frames that are temporally continuous as described above, weight information may also be assigned to the identification information. In other words, similar to when the attention position is compared with the other position in a certain spatial direction and range, at the same temporal position, the weight information defined according to at least one of the direction and distance (a time difference from the time point to be paid attention to the time point to be compared) may be assigned to the identification information. For example, point noise often blink randomly, while changing locations in a short period of time. On the other hand, blood flows often appear collectively within a certain time region in accordance with the pulsation. Therefore, in extracting a local peak position in the continuous frames, the weight information with respect to the identification information at the point of time serving as the starting point may be set to 1, while reducing the weight with respect to the identification information of the frame at the point of time temporally away from the point of time serving as the starting point to 0.8, 0.6, or the like, with an increase in time. Consequently, it is possible to efficiently suppress the point noise by using the difference in properties between the point noise and blood flow.

In the embodiment described above, the extraction of identification information, the generation of filter information, and the output of second ultrasonic data are performed on the two-dimensional ultrasonic data. However, the embodiment is not limited thereto, and the extraction of identification information, the generation of filter information, and the output of second ultrasonic data may also be performed on three-dimensional ultrasonic data. In such a case, for example, the three-dimensional ultrasonic data may also be generated, by acquiring a reception signal from the three-dimensional space, by shaking and moving a one-dimensional ultrasonic probe. Alternatively, the three-dimensional ultrasonic data may be generated, by acquiring a reception signal from the three-dimensional space by a two-dimensional ultrasonic probe. Moreover, similar to the embodiment described above, the extraction of local peak position (the extraction of identification information), the generation of filter information, and the output of second ultrasonic data may be performed, by three-dimensionally arranging the kernels with respect to the three-dimensional ultrasonic data.

The extraction of local peak position is not limited to the method described above, and may be performed using other methods. For example, the local peak position may be extracted, by performing peak sharpening that applies a nonlinear function on the signal value in the frame. In such a case, for example, the extraction function 114 first performs resampling on the first ultrasonic data to increase the pixel density, and then performs a process of increasing the effective resolution. In this example, by performing resampling on the first ultrasonic data, each pixel may be replaced with a plurality of pixels with smaller size, for example. Moreover, for example, the signal value of each pixel after being replaced may be interpolated from the original signal value (signal value before being replaced), by bi-cubic interpolation or the like.

Furthermore, the extraction function 114 sharpens the local peak, by raising each signal value of the resampled first ultrasonic data by the power (for example, the power of eight, the power of twelve, and the like). For example, the extraction function 114 extracts the local peak position, by performing threshold processing on the powered signal value and the like. In other words, the extraction function 114 extracts the identification information in which the position extracted as the local peak is represented by "1", and the position not extracted as the local peak is represented by "0". By performing the sharpening processing of the local peak described above on the first ultrasonic data, the extraction function 114 extracts the identification information from each of the first ultrasonic data. The sharpening processing of the local peak described above may also be performed on the frame data in addition to the first ultrasonic data.

Alternatively, the first ultrasonic data may be obtained, by first creating frame data with increased effective resolution, by performing resampling and increasing the pixel density, and then combining the frame data and applying the kernel. In this case, the size of the kernel to be applied is preferably increased according to the magnification to improve the effective resolution of resampling. For example, for the kernel size of (3 × 1), if the effective resolution of an image is increased by twice (that is, the resolution of an image of 512 pixels × 512 pixels is increased to the resolution of an image of 1024 pixels × 1024 pixels), the kernel size of (6 × 2) may be used.

The generation function 115 generates filter information, on the basis of the multiple pieces of identification information extracted by the sharpening processing of the local peak.

Moreover, the ultrasonic diagnostic apparatus 10 according to the present embodiment can change the scanning conditions of ultrasonic scanning when the Process 2 to the Process 4 described above are performed. Specifically, the ultrasonic diagnostic apparatus 10 can change the scanning conditions so that the line density (the number of scanning lines) is increased, when the mode is switched to the mode of performing the Process 2 to the Process 4 described above (hereinafter, referred to as a "processing application mode").

For example, when the mode of performing addition processing of the frame data to display is applied, or the normal color Doppler mode, is switched to the processing application mode, the control function 111 controls the transmission circuitry 9 and the reception circuitry 11, and causes the ultrasonic probe 5 to perform the ultrasonic scanning with an increased line density. Consequently, the reception signal with an increased number of scanning lines in the frame data is obtained. In this manner, the control function 111 sequentially acquires the frame data with a greater number of scanning lines in the frame data, compared to the mode before being switched.

Moreover, the line density may also be changed and increased in the processing application mode. For example, two modes of a first processing application mode and a second processing application mode are further applied to the processing application mode. The control function 111 may not change the line density when the mode is switched to the first processing application mode, and increase the line density when the mode is switched to the second processing application mode.

Still furthermore, in the embodiment described above, the second ultrasonic data 24 is generated and output, by multiplying the signal value representing each position in the frame represented by the first ultrasonic data 21, by the filter information (weight coefficient) set for each position corresponding to the filter information 23. However, the embodiment is not limited thereto, and the second ultrasonic data may also be generated and output, by using another mathematical formula.

For example, the output function 116 may generate and output the second ultrasonic data 24, by raising the filter information (weight coefficient) set for each position corresponding to the filter information 23, to the power of the signal value (amplitude value) representing each position in the frame represented by the first ultrasonic data 21. In other words, when the signal value of each position is "A" and the weight coefficient is "a", the output function 116 generates and outputs the second ultrasonic data 24, by calculating "A^{a}" for all the positions.

Still furthermore, in the embodiment described above, the generated filter information is multiplied by the signal value of the corresponding position (pixel) in the first ultrasonic data (in other words, one weight coefficient is multiplied by the signal value of only one pixel). However, the embodiment is not thereto, and for example, one weight coefficient may be multiplied by the signal value of the multiple pixels. To give an example, one weight coefficient may be multiplied by each signal value of 3 × 3 pixels around the pixel at the corresponding position.

Still furthermore, in the embodiment described above, in generating the filter information, the weight coefficient is calculated according to the number of times "1" has appeared at the positions corresponding to each other in each piece of the identification information 22. However, the embodiment is not limited thereto, and the weight coefficient may be set to "1", when "1" has appeared even once in the identification information.

Moreover, in the ultrasonic diagnostic apparatus, the medical information processing apparatus, and the medical information processing method according to the present application, various techniques can be applied, in addition to the embodiment described above. Hereinafter, these techniques will be described below.

For example, in the ultrasonic diagnostic apparatus, the medical information processing apparatus, and the medical information processing method according to the present application, the process of deleting an irrelevant signal point and deleting irrelevant frame data disclosed in Japanese Patent Application No. 2023-164256 may be applied. In such a case, for example, the extraction function 114 extracts an irrelevant signal point in the frame data, on the basis of the signal values of the frame data. For example, at step S104 in FIG. 2, the acquisition function 113 may acquire the first ultrasonic data of the subject, by performing addition processing of the frame data, after deleting the irrelevant signal point extracted by the extraction function 114.

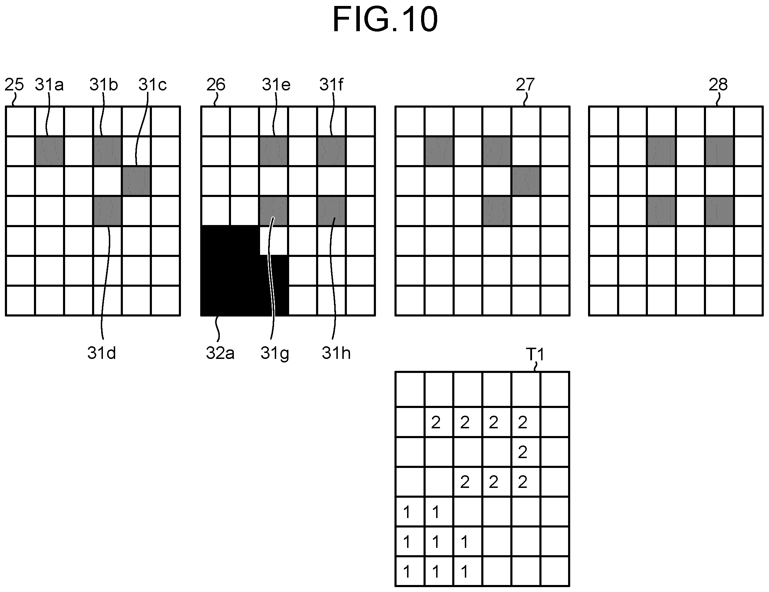

Hereinafter, an example of processing performed by the extraction function 114 will be described with reference to FIG. 10. In this example, frame data 25 to 28 illustrated in FIG. 10 represent the frame data obtained after the process at step S103 in FIG. 2 is performed. For example, the extraction function 114 performs local peak extraction processing on the frame data 25 to 28, and extracts the pixels (pixels illustrated in gray or black in the drawings) indicating the signals in which the signal value is the local maximum value. For the local peak extraction processing, the process used for extracting the local peak position described in the embodiment of the present application may be used as appropriate.

Next, the extraction function 114 extracts an irrelevant signal point, on the basis of the results of the local peak extraction processing. In this example, the irrelevant signal point is a signal point not considered as a blood flow signal to be observed, such as clutter, for example. It is considered that the clutter or the like is unlikely to be extracted as a high signal point across the frames at the same position. Hence, the extraction function 114 specifies the irrelevant signal point on the basis of the number of signal extraction times across the frame data. For example, the extraction function 114 specifies the signal point at the position where the number of signal extraction times is less than the reference, as the irrelevant signal point.

To give an example, as illustrated in FIG. 10, the extraction function 114 generates a table T1 in which the number of times each of the points in the frame data 25 to 28 is extracted as the signal point at the local peak is counted. In this example, pixels 31a, 31b, 31c, and the like illustrated in gray are extracted as local peaks in two or more frame data. Hence, the number of signal extraction times is "2". On the other hand, the pixels belonging to a region 32a illustrated in black are extracted as the local peaks only in frame data 26, that is, only in one frame data. Hence the number of signal extraction times is "1".

In this example, the processing circuitry 110 sets the number of signal extraction times, which is the reference, to "2", for example. In such a case, in the processing circuitry 110, a specification function 110h determines that the signal points in a region 31 that are the points 31a, 31b, 31c and the like, where the number of signal extraction times is two or more, as the relevant signal points. On the other hand, the extraction function 114 specifies the signal points in a region 32 where the number of signal extraction times is one, as the irrelevant signal points. The acquisition function 113 acquires the first ultrasonic data of the subject, by performing addition processing of the frame data 25 to 28, after deleting the signal points (signal points of the region 32a) extracted by the extraction function 114. In the example described above, determination processing of an irrelevant signal point is performed on the basis of the number of signal extraction times. However, the embodiment is not limited thereto, and the irrelevant signal point may also be specified using a neural network.

Moreover, for example, the extraction function 114 extracts irrelevant frame data in the frame data, on the basis of the signal value in the frame data. The acquisition function 113 acquires the first ultrasonic data of the subject, by performing addition processing of the frame data, after deleting the frame data extracted by the extraction function 114. In this example, the irrelevant frame data is frame data estimated to include a signal point not considered as a blood flow signal to be observed, such as clutter, for example. When viewed in the frame direction, it is considered that the number of signals extracted as the local peaks changes abruptly around the frame where clutter is generated. Hence, the extraction function 114 specifies irrelevant frame data, on the basis of the number of signals extracted in the frame direction. For example, the extraction function 114 specifies the frame data in which the changes in the number of signals extracted in the frame direction is greater than the reference, as the irrelevant frame data.

To give an example, in the frame data 26 in FIG. 10, the number of signal points extracted as the signal point at the local peak is "12". In contrast, in the frame data 25, 27 and 28, the number of signal points extracted as the signal point at the local peak is "4". In this example, if the number of signal points to be the reference is "6", the changes in the number of signals extracted in the frame direction around the frame data 26 exceeds the reference. Hence, the extraction function 114 specifies the frame data 26 as the irrelevant frame. The acquisition function 113 acquires the first ultrasonic data of the subject, by performing addition processing of the frame data 25, 27, and 28, after deleting the irrelevant frame data 26 extracted by the extraction function 114. In the example described above, the determination processing of the irrelevant frame data is performed on the basis of the changes in the number of signals extracted in the frame direction. However, the embodiment is not limited thereto. The irrelevant frame data may also be specified by the comprehensive criteria using a neural network.

As described above, the extraction function 114 extracts the identification information, by performing extraction processing of the local peak position on the first ultrasonic data that is acquired after an irrelevant signal point is deleted, or on the first ultrasonic data acquired after irrelevant frame data is deleted.

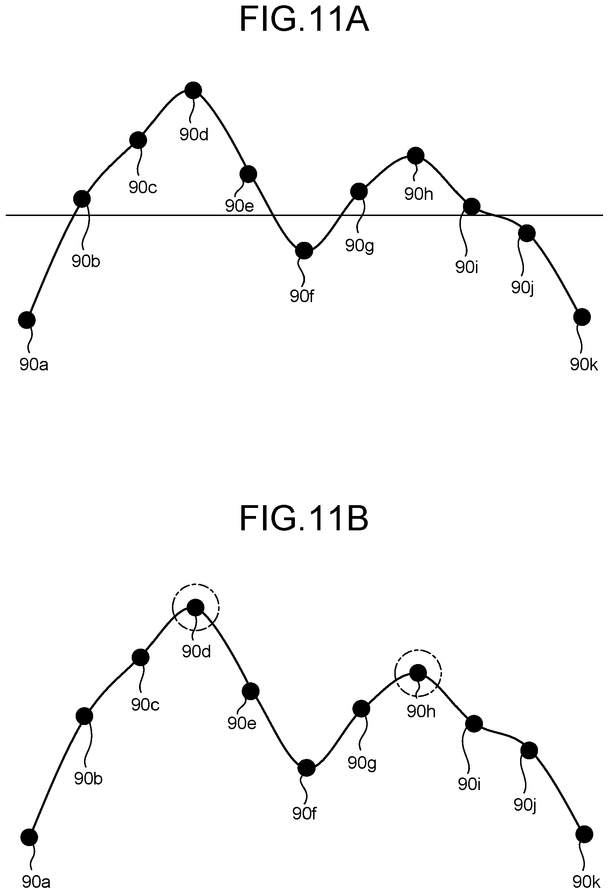

Moreover, for example, in the ultrasonic diagnostic apparatus, the medical information processing apparatus, and the medical information processing method according to the present application, the local peak position can be extracted, by applying the detection processing of the local peak disclosed in Japanese Patent Application No. 2023-164256 on the first ultrasonic data or the frame data. In such a case, for example, the extraction function 114 extracts the local peak position, by performing threshold processing or extraction processing of the local maximum value at step S105 in FIG. 2. Hereinafter, an example of the processing performed by the extraction function 114 will be described with reference to FIG. 11A and FIG. 11B. Points 90a to 90k illustrated in FIG. 11A and FIG. 11B are schematic illustrations of signal points in each signal data in a single frame, and the vertical direction on the paper surface represents the size of each signal data.

For example, as illustrated in FIG. 11A, the extraction function 114 extracts the points 90b, 90c, 90d, 90e, 90g, 90h, and 90i the signal values of which are equal to or greater than the threshold (horizontal line in the drawing) illustrated in FIG. 11A , as the local peak positions. Moreover, for example, as illustrated in FIG. 11B, the extraction function 114 extracts the points 90d and 90h that are the local maximum values, as the local peak positions.

In FIG. 11A and FIG. 11B, to simplify the explanation, the signal data is illustrated as one-dimensional signal data. However, in general, the signal data of each frame is two-dimensional signal data. Even if the signal data is the signal data of two-dimensional or more, by performing the same process as the process described above, the extraction function 114 can extract the local peak position.

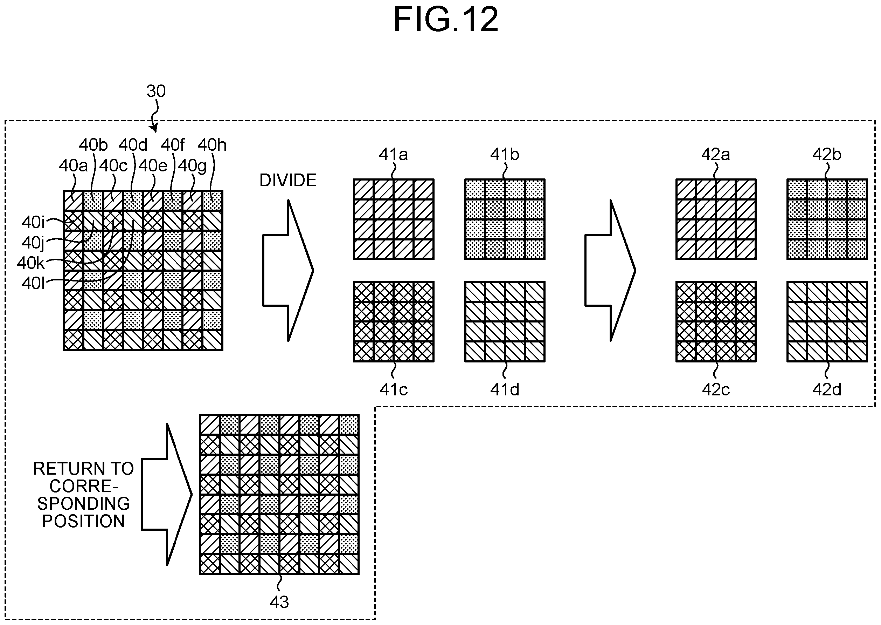

Moreover, for example, in the ultrasonic diagnostic apparatus, the medical information processing apparatus, and the medical information processing method according to the present application, the high-signal point extraction processing disclosed in Japanese Patent Application No. 2023-213406 may be applied to extract the local peak position. Hereinafter, this process will be described with reference to FIG. 12. In this example, in FIG. 12, image data 30 indicates data (first ultrasonic data or frame data) to be extracted at the local peak position. Pixels 40a, 40b, 40c, 40d, 40e, 40f, 40g, 40h, 40i, 40j, 40k, and 40l are pixels in the image data 30. For example, at step S105 in FIG. 2, the extraction function 114 performs a process of extracting identification information from the data (for example, first ultrasonic data) to be extracted at the local peak position as follows.

For example, the extraction function 114 generates four pieces of division data from the first ultrasonic data (divides the first ultrasonic data into four pieces), by performing data division processing on the image data 30 (hereinafter, may be referred to as first ultrasonic data), while thinning out the data at regular intervals. To give an example, the extraction function 114 performs data division processing on the image data 30 that is the first ultrasonic data, while skipping the data one by one vertically and horizontally. The extraction function 114 then divides the image data 30 into image data 41a including the pixels 40a, 40c, 40e, 40g, and the like illustrated in the drawing; image data 41b including the pixels 40b, 40d, 40f, 40h, and the like; image data 41c including the pixels 40i, 40k, and the like; and image data 41d including the pixels 40j, 40l, and the like. In other words, the extraction function 114 performs the division of 2 in height × 2 in width and dividing the image data 30 that is the first ultrasonic data into two vertical and horizontal parts. Hence, four image data (division data) 41a, 41b, 41c, and 41d are generated from the first ultrasonic data.

In the example described above, the image data 30 is divided into four first ultrasonic data, by performing the division of 2 in height × 2 in width and dividing the image data 30 into two vertical and horizontal parts. However, the embodiment is not limited thereto. For example, the image data may also be divided into nine pieces of first ultrasonic data, by performing the division of 3 in height × 3 in width and dividing the image data into three vertical and horizontal parts. Alternatively, the image data may also be divided into 16 pieces of first ultrasonic data, by performing the division of 4 in height × 4 in width and dividing the image data into four vertical and horizontal parts. Moreover, for example, the image data may be divided into two pieces of first ultrasonic data, by performing the division of 2 in height × 1 in width and dividing the image data into two vertical parts only. Alternatively, the image data may also be divided into two first ultrasonic data, by performing the division of 1 in height × 2 in width and dividing the image data into two horizontal parts only.

Subsequently, the extraction function 114 extracts the local peak position from each of the division data. For the peak extraction processing, the process used for extracting the local peak position disclosed in the embodiment of the present application may be used as appropriate. The extraction function 114 extracts division identification information 42a, 42b, 42c, and 42d that indicate the extraction results of the local peak positions, from each of the division data.

The extraction function 114 generates image data 43, by allocating the division identification information 42a, 42b, 42c, and 42d to the corresponding positions on the first ultrasonic data. The image data 43 is the extraction result of the position of the high signal point, and corresponds to the identification information.

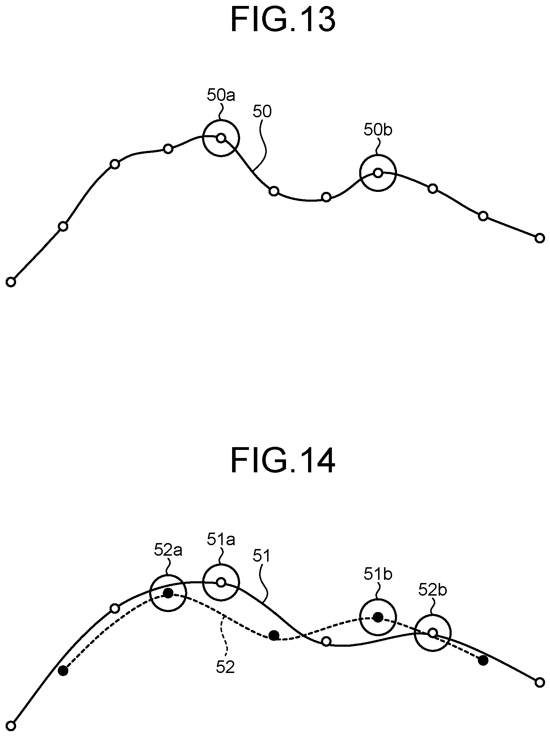

The effects of the high-signal point extraction processing described above will be explained with reference to FIG. 13 and FIG. 14. FIG. 13 is a conceptual diagram illustrating a high-signal point extraction method according to a comparative example. FIG. 8 is a conceptual diagram illustrating a high-signal point extraction method according to another embodiment.

FIG. 13 illustrates a high-signal point extraction method according to a comparative example, and illustrates an extraction method that performs high-signal point extraction without performing space division. Each point in FIG. 13 represents each signal point, and a curve 50 is obtained by connecting the signal values. As illustrated in FIG. 13, when signal extraction is performed without performing the space division, a signal point 50a and a signal point 50b are extracted as the positions of the high-signal points.

In contrast, FIG. 14 illustrates a high-signal point extraction method according to another embodiment, and illustrates an extraction method that performs high-signal point extraction by performing space division. Each point in FIG. 14 represents each signal point, and a curve 51 and a curve 52 are obtained by connecting the signal values in each image data on which division processing is performed. When signal extraction is performed on each image data on which division processing is performed, signal points 51a and 51b, and signal points 52a and 52b are extracted as the positions of the high signal points. In other words, by using the method according to another embodiment, it is possible to extract a highly bright signal region in a wider range, compared to the comparative example. In other words, with the method of another embodiment, an image component representing an object is continuously extracted. Hence, it is possible to continuously extract the peak pixel values. The peak in the attention position is determined basically in pixel units. However, the peak may also be determined in group units including multiple pixels. When the peak is determined in group units, the pixel value of the group may be the average value or the integrated value of the pixel values of the pixels in a plurality of groups.

Still furthermore, in the embodiment described above, the identification information at each position in the frame represented by the first ultrasonic data is extracted. However, the embodiment is not limited thereto, and for example, identification information may only be extracted from the position in some region of the first ultrasonic data. In other words, in a predetermined range of the frame, the extraction function 114 extracts the information that identifies the position having a higher signal value than that of the surrounding area, as the identification information.

Still furthermore, in the embodiment described above, the processing using the identification information is performed on at least one of the first ultrasonic data or on ultrasonic data obtained by performing a predetermined process on the first ultrasonic data. However, the embodiment is not limited thereto, and the ultrasonic data representing an object to be processed may also be other than at least one of the first ultrasonic data and the ultrasonic data obtained by performing a predetermined process on the first ultrasonic data.

Still furthermore, the processing of deleting the irrelevant signal point and deleting the irrelevant frame data, the detection processing of local peak, and the extraction processing of high luminance point described above may be combined, or the process to be used may be selected, by the imaging target, the imaging mode, or the input processing performed by the user. For example, the extraction processing of high luminance point may be applied, when the imaging mode is shifted to a mode in which the effective resolution of frame data is fine to a certain degree or more.

Moreover, for example, in the ultrasonic diagnostic apparatus, the medical information processing apparatus, and the medical information processing method according to the present application, the kernel disclosed in Japanese Patent Application No. 2023-213405 may be used to extract the local peak position. Specifically, a kernel in which only the square at the limited position among the squares in the kernel may be used for extracting the local peak position. For example, the extraction function 114 extracts the local peak position, by using a filter that only extracts the first, third, and fifth squares from left among the squares of 5 × 1, that are the kernels of 5 × 1, as the kernel, and by comparing the extracted signal value with the adjacent signal value. To give an example, the extraction function 114 extracts the local peak position, by setting the position of the third square as the attention position, and setting the positions of the first and fifth squares as the surrounding areas, and comparing the signal values of the squares.

## Claims

1. An ultrasonic diagnostic apparatus (10), comprising:
an ultrasonic probe (5) configured to transmit an ultrasonic wave to a subject, and receive the ultrasonic wave reflected by the subject;
an acquisition unit (113) configured to acquire first ultrasonic data of the subject, based on the ultrasonic wave received by the ultrasonic probe (5);
an extraction unit (114) configured to extract identification information that identifies a position of a local peak for each predetermined region of a frame represented by the first ultrasonic data; and
an output unit (116) configured to output second ultrasonic data, based on the identification information extracted by the extraction unit (114), and ultrasonic data representing an object to be processed.

2. The ultrasonic diagnostic apparatus (10) according to claim 1, wherein the extraction unit (114) is configured to extract the identification information, by identifying a position where a signal value is relatively high as the position of the local peak for each local region of the frame represented by the first ultrasonic data.

3. The ultrasonic diagnostic apparatus (10) according to claim 1, wherein
the acquisition unit (113) is configured to acquire a plurality of the first ultrasonic data within a predetermined time,
the extraction unit (114) is configured to extract the identification information that identifies the position of the local peak, for each frame represented by the first ultrasonic data, and
the output unit (116) is configured to output the second ultrasonic data, based on the identification information extracted for each of the frames, and the ultrasonic data.

4. The ultrasonic diagnostic apparatus (10) according to claim 1, wherein at each position in the frame, the extraction unit (114) is configured to extract information that identifies a magnitude relation between a signal value corresponding to the position and a signal value corresponding to a surrounding area of the position, as the identification information.

5. The ultrasonic diagnostic apparatus (10) according to claim 4, wherein at each position in the frame, the extraction unit (114) is configured to extract information that identifies whether the signal value corresponding to the position is greater than the signal value corresponding to the surrounding area of the position, as the identification information.

6. The ultrasonic diagnostic apparatus (10) according to claim 4, wherein the surrounding area is a position in a spatial direction and range with respect to the position, or a position in a temporal direction and range with respect to the position.

7. The ultrasonic diagnostic apparatus (10) according to claim 1, wherein in a predetermined range of the frame, the extraction unit (114) is configured to extract information that identifies a position having a higher signal value than that of a surrounding area, as the identification information.

8. The ultrasonic diagnostic apparatus (10) according to claim 7, wherein the surrounding area is a position in a spatial direction and range with respect to the position, or a position in a temporal direction and range with respect to the position.

9. The ultrasonic diagnostic apparatus (10) according to claim 1, wherein at each position in the frame represented by the first ultrasonic data, the extraction unit (114) is configured to extract the identification information.

10. The ultrasonic diagnostic apparatus (10) according to claim 1, wherein the ultrasonic data representing the object to be processed is, at least one of the first ultrasonic data, or ultrasonic data obtained by performing a predetermined process on the first ultrasonic data.

11. The ultrasonic diagnostic apparatus (10) according to claim 1, comprising:
a generation unit (115) configured to generate filter information corresponding to each position in the frame, based on the identification information extracted from each of the first ultrasonic data by the extraction unit (114); wherein
the output unit (116) is configured to output the second ultrasonic data, based on the filter information and the ultrasonic data representing the object to be processed.

12. The ultrasonic diagnostic apparatus (10) according to claim 11, wherein the extraction unit (114) is configured to assign weight information defined according to each of the first ultrasonic data to the identification information.

13. The ultrasonic diagnostic apparatus (10) according to claim 5, wherein
the surrounding area with which the magnitude relation between the signal values is identified by the extraction unit (114), is a position in a certain spatial direction and distance with respect to the position, or a position in a certain temporal direction and distance with respect to the position, and
the extraction unit (114) is configured to assign weight information defined according to at least one of the direction and distance, to the identification information.

14. The ultrasonic diagnostic apparatus (10) according to claim 1, wherein the acquisition unit (113) is configured to acquire addition data that is obtained by performing ultrasonic scanning on a subject, and that is obtained by performing addition processing of a plurality of frame data continuous in a temporal direction, as the first ultrasonic data.

15. The ultrasonic diagnostic apparatus (10) according to claim 10, wherein at each position in the frame represented by the first ultrasonic data, the output unit (116) is configured to set ultrasonic data obtained by performing a process of extracting a signal value corresponding to the position, based on a magnitude relation between a signal value corresponding to the position and a signal value corresponding to a surrounding area of the position, as the ultrasonic data obtained by performing the predetermined process, and output the second ultrasonic data, based on the ultrasonic data and the identification information.

16. A medical information processing method, comprising:
acquiring first ultrasonic data of a subject;
extracting identification information that identifies a position of a local peak for each predetermined region of a frame represented by the first ultrasonic data; and
outputting second ultrasonic data, based on the identification information extracted by the extracting, and ultrasonic data representing an object to be processed.
